# EUROPEAN PATENT APPLICATION

(11) **EP 3 701 850 A1**
(43) Date of publication of application: **02.09.2020**
(21) Application number: 19763774.7
(22) Date of filing: 04.03.2019
(51) Int. Cl.: A61B 1/00, A61B 1/045, A61B 1/06, A61B 1/12

(54) **APPLICATOR DEVICE FOR ENDOSCOPE**

(30) Priority: 05.03.2018 JP 2018038766
(71) Applicant: Goryo Chemical, Inc., Sapporo-shi, Hokkaido 060-0008 (JP)
(72) Inventor: YOSHIDA, Takashi, Hachioji-shi, Tokyo 192-0046 (JP)
(74) Representative: Blumbach · Zinngrebe Patentanwälte PartG mbB
(86) International application number: PCT/JP2019/008428
(87) International publication number: WO 2019/172189

(57) **Abstract**

Provided is an applicator device (1) for an endoscope, including an applicator (42) attachable to and removable from an insertion part (16) of an endoscope (4) to be inserted into a body cavity, and the applicator (42) comprises a light emitting element (64) that irradiates an affected area of a biological tissue in the body cavity with excitation light that excites a photosensitive substance administered to the affected area so that the photosensitive substance acts on the affected area.

## Description

### Technical Field

The present invention relates to an applicator device for an endoscope, and in particular to an applicator device for an endoscope that is applicable to an endoscope system that performs an endoscopic treatment.

### Background Art

Patent Document 1 discloses an endoscope with a hood that is capable of irradiating a body cavity with exciting laser light through an optical fiber protruding from a forceps opening made in a tip portion of an insertion part of the endoscope.

Furthermore, Patent Document 2 discloses an electronic endoscope that is capable of irradiating a body cavity with exciting laser light through an optical fiber disposed in an overtube mounted to a tip hood of an insertion part of the endoscope.

### Prior Art Document

### Patent Document

Patent Document 1: Japanese Patent Laid-Open No. 2012-50511
Patent Document 2: Japanese Patent Laid-Open No. 2012-81048

### Summary of the Invention

### Problems to be solved by the Invention

In Patent Document 1, however, a forceps opening of an endoscope is for use in irradiation with laser light. During the use, therefore, the optical fiber occupies the forceps opening, and a treatment instrument cannot be used through the forceps opening in the endoscope. Furthermore, during the irradiation with the laser light and during a treatment, a switching work of inserting and removing the optical fiber and the treatment instrument into and from the endoscope occurs. Consequently, an operation associated with use of the endoscope is laborious, and the work requires much time.

Furthermore, in Patent Document 2, both a tip hood and an overtube need to be mounted to an endoscope. Consequently, a configuration of the endoscope is complicated, and a work is laborious.

The present invention has been developed in view of such problems, and an object of the present invention is to provide an applicator device for an endoscope that is capable of smoothly performing an endoscopic treatment.

### Means for Solving the Problems

The present invention can be achieved as the following aspect.

An applicator device for an endoscope according to the present aspect is an applicator device for an endoscope including an applicator attachable to and removable from an insertion part of the endoscope to be inserted into a body cavity, and the applicator comprises a light emitting element that irradiates an affected area of a biological tissue in the body cavity with excitation light that excites a photosensitive substance administered to the affected area so that the photosensitive substance acts on the affected area.

Furthermore, a plurality of light emitting elements described above according to the present aspect are arranged along a circumferential direction of the applicator.

Additionally, the applicator according to the present aspect comprises a channel to administer the photosensitive substance to the biological tissue in the body cavity.

Furthermore, a plurality of channels described above according to the present aspect are opened along a circumferential direction of the applicator.

Additionally, the endoscope according to the present aspect includes, in a tip portion of the insertion part, an illuminating section that illuminates the body cavity with illumination light, a light receiving section that receives light from the body cavity, and a forceps opening that a treatment instrument inserted into the insertion part enters and exits.

Furthermore, the applicator according to the present aspect comprises an optical filter that covers the light receiving section to dim or cut off at least the excitation light.

Additionally, the applicator according to the present aspect includes filter adjustment means for adjusting an amount of the excitation light to be dimmed or switching whether to cut off the excitation light, by the optical filter.

Furthermore, the above described endoscope according to the present aspect is included in an endoscope system comprising a light source device that generates illumination light to guide the light to the illuminating section, a processor device that processes a captured image based on light received from the light receiving section to generate an observation image, and a monitor device electrically connected to the processor device, to display the observation image generated by the processor device, and the applicator device for the endoscope further comprises a power source device that supplies power to the light emitting element and is electrically connected to the processor device, and control means for attenuating or cutting off an output signal concerning the excitation light from the processor device to the monitor device, when the power source device supplies the power to the light emitting element.

Additionally, the applicator according to the present aspect is a tip hood mounted to a tip portion of the insertion part.

Furthermore, the photosensitive substance according to the present aspect is a luminescent probe, and the light emitting element irradiates the luminescent probe administered to the affected area of the biological tissue in the body cavity by the applicator with excitation light that excites the luminescent probe to emit light.

The applicator device for the endoscope further comprises supply start means for starting supplying the luminescent probe through the channel according to the present aspect, irradiation start means for starting the irradiation with the excitation light from the light emitting element, and determination means for determining whether a residual affected area is present in the biological tissue, based on a result of luminescence observation of the luminescent probe with the light received from the light receiving section, when after treating the affected area in the biological tissue with the treatment instrument, starting supplying the luminescent probe by the supply start means, and starting the irradiation with the excitation light by the irradiation start means.

Furthermore, the luminescent probe according to the present aspect is a fluorescent probe.

Additionally, the light emitting element according to the present aspect irradiates the affected area of the biological tissue in the body cavity with the excitation light that excites the photosensitive substance accumulated in the affected area to cause damage to the affected area by a photochemical reaction.

In addition, the light emitting element according to the present aspect is a light emitting diode.

### Advantageous Effects of the Invention

According to the above described aspect of an applicator device for an endoscope of the present invention, an endoscopic treatment can be smoothly performed.

### Brief Description of the Drawings

FIG. 1 is a schematic view of an endoscope system to which an applicator device for an endoscope according to an embodiment of the present invention is applied.
FIG. 2 is a front view showing a tip portion in an insertion part of the endoscope in FIG. 1.
FIG. 3 is a perspective view showing a state before an applicator is mounted to the tip portion of the endoscope in FIG. 2.
FIG. 4 is a perspective view showing a state after the applicator is mounted to the tip portion of the endoscope from the state in FIG. 3.
FIG. 5 is a cross-sectional view taken along the A-A line in FIG. 4.
FIG. 6 is a perspective view showing a state before an applicator according to another embodiment of the present invention is mounted to the tip portion of the endoscope in FIG. 2.
FIG. 7 is a perspective view showing a state before an applicator according to still another embodiment of the present invention is mounted to the tip portion of the endoscope in FIG. 2.

### Mode for Carrying out the Invention

Hereinafter, description will be made as to an applicator device for an endoscope according to an embodiment of the present invention with reference to the drawings.

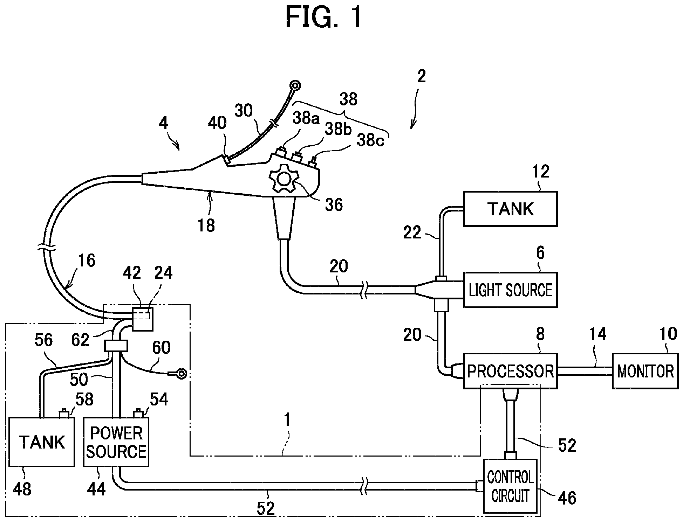

FIG. 1 shows a schematic view of an endoscope system 2 to which an applicator device 1 for an endoscope of the present embodiment (hereinafter, also referred to simply as the applicator device 1) is applied. The endoscope system 2 comprises an endoscope 4, a light source device 6 electrically and optically connected to the endoscope 4, a processor device 8 electrically connected to the endoscope 4, a monitor device 10 electrically connected to the processor device 8 via a cable 14, and an air/water-feeding tank 12 in which air and water to be supplied to the endoscope 4 are stored.

The endoscope 4 comprises an insertion part 16 having flexibility and configured to be inserted into a body cavity of a subject, an operation unit 18 coupled to a base end portion of the insertion part 16, a universal cord 20 extending from the operation unit 18, and an air/water-feeding tube 22. The universal cord 20 contains unshown cords electrically connecting the operation unit 18 to the light source device 6 and the processor device 8, respectively. The air/water-feeding tube 22 is provided to feed air and water from the air/water-feeding tank 12 toward the operation unit 18, and the universal cord 20 contains a part of the air/water-feeding tube 22.

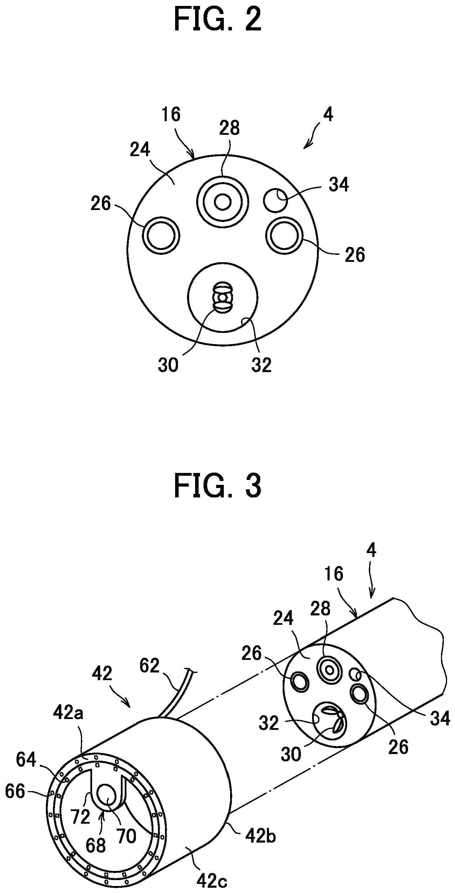

FIG. 2 shows a front view of a tip portion 24 in the insertion part 16 of the endoscope 4. The tip portion 24 is formed with two illuminating sections 26 that illuminate the body cavity with illumination light, a light receiving section 28 that receives light from the body cavity, a forceps opening 32 that a treatment instrument 30, such as a pair of forceps, inserted into the insertion part 16 enters and exits, and an air/water-feeding port 34 to feed air and water into the body cavity.

In the insertion part 16 and the operation unit 18 shown in FIG. 1, although neither is shown in the drawing, there are provided a forceps channel in which the treatment instrument 30 is inserted toward the forceps opening 32 and an air/water-feeding channel through which air and water flow from the air/water-feeding tank 12 toward the air/water-feeding port 34. Each of the illuminating sections 26 illuminates the body cavity with the illumination light from the tip portion 24 through an unshown optical fiber inserted into the operation unit 18 and the insertion part 16, the illumination light being generated in the light source device 6.

The light receiving section 28 comprises, for example, an objective lens and an imaging element that are not shown in the drawing, and receives the light from the body cavity to acquire information of a captured image of an observation target. The light source device 6 comprises an unshown light source such as a xenon lamp or a halogen lamp, and generates the illumination light by a light source, for example, based on an operation of the operation unit 18. This illumination light is, for example, white light, and guided to each of the illuminating sections 26 of the tip portion 24 through the universal cord 20, the operation unit 18 and the insertion part 16 in this order. Note that the light source device 6 may include another light source.

The processor device 8 processes the information of the captured image obtained by the light receiving section 28 to generate an observation image, for example, based on the operation of the operation unit 18, and transmits the image to the monitor device 10 through the cable 14. The monitor device 10 displays the observation image generated by the processor device. Note that the monitor device 10 may be provided with an unshown input unit capable of inputting, for example, display setting of the observation image.

As shown in FIG. 1, the operation unit 18 is provided with an angle knob 36 to three-dimensionally bend the tip portion 24 of the insertion part 16, various types of operation buttons 38 including an illumination button 38a, an air/water-feeding button 38b and an imaging button 38c, and an insertion port 40 for the treatment instrument 30. The illumination button 38a is operated, so that each of the illuminating sections 26 can illuminate the body cavity with the illumination light generated in the light source device 6, through the universal cord 20, the operation unit 18 and the insertion part 16 in this order.

The air/water-feeding button 38b is operated, so that the air and water of the air/water-feeding tank 12 can be injected into the body cavity from the air/water-feeding port 34 through the air/water-feeding tube 22, the universal cord 20, the operation unit 18 and the insertion part 16 in this order. The imaging button 38c is operated, so that the information of the captured image obtained by the light receiving section 28 can be transmitted to the processor device 8 through the insertion part 16, the operation unit 18 and the universal cord 20 in this order, and the observation image displayed in the monitor device 10 can be enlarged and reduced. Furthermore, the treatment instrument 30 protruded from the insertion port 40 is operated so that an affected area in the body cavity can be treated.

In this way, the operation unit 18 can collectively perform control and operations such as control associated with the illuminating sections 26 and the light receiving section 28, an operation of the treatment instrument 30 that enters or exits through the forceps opening 32, and various operations in the tip portion 24, including the air and water feeding through the air/water-feeding port 34. Note that the endoscope 4 and the endoscope system 2 that have been described hitherto do not have such special and expensive dedicated use specifications including a highly sensitive CCD camera or the like to perform fluorescence observation, but have normal and comparatively inexpensive specifications that enable general body cavity observation and a treatment.

Here, the applicator device 1 of the present embodiment can be separately and later mounted to and used in the endoscope 4 and the endoscope system 2 having the above described normal specifications. As shown in FIG. 1, the applicator device 1 comprises an applicator 42 attachable to and removable from the tip portion 24 of the endoscope 4, a power source device 44, a control circuit (control means) 46, and a probe tank 48.

The power source device 44 is electrically connected to the applicator 42 and the processor device 8 via cables 50, 52, respectively. Furthermore, the power source device 44 is provided with a power source switch 54 capable of supplying power to an applicator 42 side via the cable 50. The control circuit 46 is electrically interposed in the cable 52 between the power source device 44 and the processor device 8.

The probe tank 48 stores a fluorescent probe (a luminescent probe or a photosensitive substance) to be supplied to the applicator 42, and is connected to the applicator 42 via a probe tube bundle 56. Furthermore, the probe tank 48 is provided with a supply switch 58 that starts supplying the fluorescent probe to the applicator 42 side via the probe tube bundle 56.

The fluorescent probe of the present embodiment is a fluorescent substance that initially has no fluorescence, but peculiarly reacts with a predetermined enzyme that excessively develops in the affected area of the biological tissue, e.g., on a membrane surface of a cancer cell, to noticeably increase the fluorescence. This fluorescent probe is irradiated with excitation light of a predetermined excitation wavelength, to generate green fluorescence. Furthermore, this fluorescent probe has an excellent cell membrane permeability.

Furthermore, the applicator 42 is connected to an adjustment wire 60 of an optical filter 68 that will be described later. Preferably, as shown in FIG. 1, the cable 50, the probe tube bundle 56 and the adjustment wire 60 are collectively connected to an end face 42b of the applicator 42 by a universal cord 62 in which the above cable and others are integrally formed.

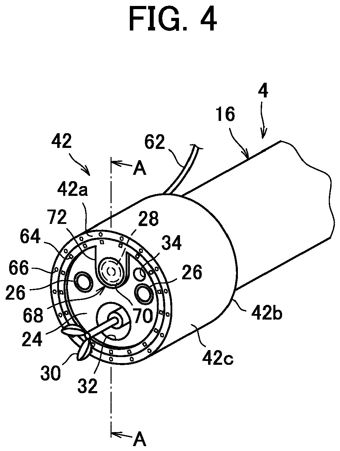

FIG. 3 shows a perspective view of a state before the applicator 42 is mounted to the tip portion 24 of the endoscope 4. Furthermore, FIG. 4 shows a perspective view of a state after the applicator 42 is mounted to the tip portion 24 of the endoscope 4. The applicator 42 possesses, for example, a cylindrical shape and is formed of a resin having flexibility to be easily attachable to and removable from the tip portion 24. Additionally, the applicator 42 is preferably formed of a transparent resin, for example, a silicone resin to minimize optical effects during normal use of the endoscope 4 and during fluorescence observation by the applicator device 1.

As shown in FIG. 4, in the applicator 42 mounted to the tip portion 24, at least the respective illuminating sections 26, the forceps opening 32 and the air/water-feeding port 34 can be exposed in the body cavity. Consequently, also in a state where the applicator 42 is mounted to the tip portion 24, the treatment instrument 30 can enter and exit through the forceps opening 32, and another function of the endoscope 4 is not restricted.

Furthermore, the applicator 42 of the present embodiment also serves as a so-called tip hood, and the applicator mounted to the tip portion 24 can prevent the tip portion 24 from coming in contact with the body cavity and thereby causing any damage or the like to the biological tissue during endoscopic diagnosis. Furthermore, the applicator 42 also plays a role of securing an appropriate distance between the tip portion 24 and the biological tissue that is the observation target, and enables appropriate illumination of the body cavity by the respective illuminating sections 26 and appropriate imaging by the light receiving section 28.

Here, in a tip surface 42a of the applicator 42 located on a tip portion 24 side of the endoscope 4, a large number of LED chips (light emitting elements or light emitting diodes) 64 are provided along a circumferential direction of the tip surface. Furthermore, on a circumferential side of each of the LED chips 64 in the tip surface 42a, a large number of channels 66 are opened along the circumferential direction of the tip surface 42a.

Each of the channels 66 is formed to extend through a circumferential portion 42c of the applicator 42 from the tip surface 42a to the end face 42b opposite to the tip surface 42a in a cylinder height direction of the applicator 42. Unshown respective probe tubes constituting the probe tube bundle 56 arranged in the universal cord 62 are connected to openings of the respective channels 66 in the end face 42b, respectively.

The supply switch 58 of the probe tank 48 is turned on, and the fluorescent probe is thereby administered from the probe tank 48 through the probe tube bundle 56 and the openings of the respective channels 66 to the biological tissue in the body cavity. Preferably, the opening of each of the channels 66 is narrowed like a nozzle, and accordingly, the fluorescent probe is evenly sprayed toward the biological tissue.

Each of the LED chips 64 is a microscopic chip capable of emitting high-luminance light, and irradiates the fluorescent probe with the excitation light that excites the probe to fluoresce. The excitation light emitted from the LED chips 64 is, for example, blue light having a predetermined excitation wavelength (e.g., about 488 nm or more). When the fluorescent probe reacts with the affected area, the probe is excited by the above described excitation light, and generates green fluorescence having an extremely large fluorescence intensity (of about 350 times the intensity prior to the reaction) in the predetermined excitation wavelength (e.g., about 525 nm). This green fluorescence is easily distinguished, for example, from the biological tissue in the fluorescence observation or from bleeding during the treatment, and is therefore suitable for specifying the affected area.

Furthermore, the applicator 42 of the present embodiment comprises the optical filter 68. The optical filter 68 is an excitation light cut filter, and as shown in FIG. 4, formed in a region that covers the light receiving section 28 from the tip surface 42a of the applicator 42 toward a center of the applicator 42 in a cylinder radial direction. The optical filter 68 comprises an optical element 70, a cover 72 that stores the optical element 70, and the adjustment wire 60 (see FIG. 1) partially contained in the universal cord 62. The adjustment wire 60 is connected to the optical element 70.

The optical element 70 is formed of, for example, a film-like glass material having a laminate-coated surface, has flexibility, and has a performance of transmitting no excitation light having the predetermined excitation wavelength or being hard to transmit the light. Consequently, the optical element 70 dims or cuts off the excitation light received by the light receiving section 28, so that the light receiving section 28 can more clearly receive the fluorescence generated by the fluorescent probe, and the fluorescence observation can be highly accurately performed. Note that in general, a fluorescence wavelength is longer than the excitation wavelength, and hence the optical filter 68 is preferably a so-called long pass filter.

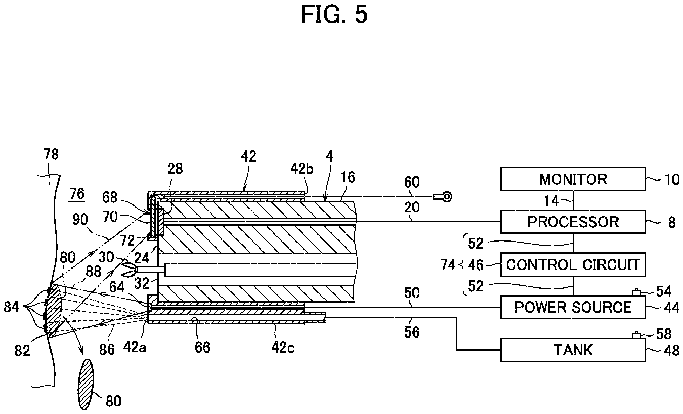

FIG. 5 is a cross-sectional view taken along the arrow A-A line in FIG. 4. Note that in FIG. 5, the treatment instrument 30 is not shown in a cross section, and a part of a configuration is not shown for description or for clarity. The cover 72 of the optical filter 68 is, for example, formed integrally with the applicator 42 by use of a resin similar to the resin of the applicator 42, and movably stores the optical element 70 along the cylinder radial direction and cylinder height direction of the applicator 42, while partially holding a circumferential portion of the optical element 70.

The adjustment wire 60 has one end connected to the optical element 70, and the other end inserted into a circumferential portion of the applicator 42 and protruded from the end face 42b of the applicator 42 through the universal cord 62. The adjustment wire 60 is removed from or inserted into the applicator 42, to operate the wire outside the applicator 42.

When the optical element 70 is moved forward to a position to cover the light receiving section 28, the optical filter 68 is operated, and when the optical element 70 is retracted to a position to expose the light receiving section 28, the optical filter 68 is not operated. Consequently, the optical filter 68 adjusts an amount of the excitation light to be dimmed or switches whether to cut off the excitation light (filter adjustment means).

At start of power supply from the power source device 44 to the respective LED chips 64, the control circuit 46 performs control to attenuate or cut off an output signal of the excitation light from the processor device 8 to the monitor device 10. Specifically, the information of the captured image obtained by the light receiving section 28 is transmitted to the processor device 8 through the universal cord 20. The cable 14 connects the processor device 8 and the monitor device 10 at so-called RGB terminals. Consequently, the information of the captured image is decomposed into analog signals of three colors of R (red), G (green), and B (blue) in the processor device 8, and transmitted to the monitor device 10.

When the power source switch 54 receives an on-signal through the cable 52, the control circuit 46 attenuates or cuts off a signal of the excitation light that is the blue light to be output from the processor device 8 to the monitor device 10, that is, an output signal from the B-terminal of the processor device 8. Thus, the control circuit 46 connected to the power source device 44 is connected to the processor device 8 of the endoscope system 2 via the cable 52, thereby making it possible to provide the existing endoscope system 2 with a so-called excitation light signal control system 74.

Hereinafter, description will be made as to an application example of the applicator device 1 to the endoscope system 2 with reference to FIG. 5. First, there is assumed a case where the endoscope system 2 is operated in the operation unit 18 to incise an affected area 80 of the cancer cell or the like that is present in a biological tissue 78 in a body cavity 76 with the treatment instrument 30 extending from the forceps opening 32, and to retract the area in an arrow direction in FIG. 5, and then residual affected areas 84 are still present on an inner side of an incised surface 82 of the incised biological tissue 78.

Based on this assumption, next in the applicator device 1 beforehand mounted to the endoscope system 2, the supply switch 58 of the probe tank 48 is turned on (supply start means). Consequently, the fluorescent probe is supplied from the probe tank 48 to the respective channels 66 through the probe tube bundle 56, and a fluorescent probe 86 (shown by broken arrow lines) is evenly sprayed from openings of the respective channels 66 to the incised surface 82 of the biological tissue 78. The fluorescent probe 86 has an excellent cell membrane permeability, and hence penetrates from the incised surface 82 of the biological tissue 78 to the residual affected areas 84, to selectively react with the residual affected areas 84.

Next, the power source switch 54 of the power source device 44 is turned on (irradiation start means). Consequently, the power source device 44 supplies power to the respective LED chips 64 through the cable 50, and the respective LED chips 64 emit excitation light 88 (shown by a one-dot chain line arrow). The fluorescent probe 86 reacted with the residual affected areas 84 is excited by the excitation light 88 to generate fluorescence 90 (shown by a two-dot chain line arrow). The captured image obtained as a result of the fluorescence 90 received by the light receiving section 28 is processed into the observation image by the processor device 8, and transmitted to the monitor device 10. By the fluorescence observation of this observation image, it can be easily determined whether the residual affected areas 84 are present (determination means).

As above, the applicator device 1 of the present embodiment is configured as a device in which functions specialized in the fluorescence observation are only integrated, and the forceps opening 32 is not restricted by a use application other than use of the treatment instrument 30. Specifically, in the fluorescence observation and subsequent treatments, it is not necessary to attach or remove the treatment instrument 30 to or from the endoscope 4 or to attach or remove the insertion part 16 of the endoscope 4 to or from the body cavity. Thus, the applicator device 1 does not interfere with basic functions of the endoscope system 2 and the endoscope 4, so that an endoscopic treatment in performing the fluorescence observation can be smoothly performed.

Furthermore, for example, in case where the affected area is treated by an endoscopic submucosal dissection method (ESD method) through the endoscope 4, heretofore, an incised piece has been pathologically judged, followed by infiltration judgment for the cancer cell. If it is determined that the cancer cell remains, it is necessary to perform an endoscopic surgical operation anew on another day. However, when the applicator device 1 is applied to the endoscope system 2, a treatment, fluorescence observation, determination of a next treatment policy and a retreatment can be all rapidly performed in this one endoscopic surgical operation. Therefore, the endoscopic treatment in performing the fluorescence observation can be further smoothly performed.

In more detail, the applicator 42 provided with the respective LED chips 64 is removably provided in the tip portion 24 of the insertion part 16 of the endoscope 4, so that the respective LED chips 64 of the applicator 42 can emit the excitation light. Therefore, any optical fiber to emit exciting laser light does not have to be protruded from the forceps opening 32, and an overtube or the like to dispose the optical fiber or an expensive laser light emitting device is not required.

Therefore, it is possible in the fluorescence observation to excite the fluorescent probe to fluoresce with a simple configuration and at low cost. Note that in recent years, the LED chips 64 have had their luminance enhanced. Furthermore, the fluorescent probe of the present embodiment can obtain an extremely large fluorescence intensity even from low-output excitation light, and this is why each of the LED chips 64 is applied as an excitation light source to the applicator 42 for the endoscope 4.

Furthermore, a large number of LED chips 64 are arranged along a circumferential direction of the applicator 42, so that fluorescence generated in the affected area can be evenly excited with high illuminance. Consequently, a clearer observation image can be obtained.

Additionally, the channel 66 is provided in the applicator 42 so that the fluorescent probe is administered through the channel 66, and hence the forceps opening 32 does not have to be used to administer the fluorescent probe. Furthermore, for example, to perform the fluorescence observation after the treatment, a method of inserting a tube into the body cavity to supply the fluorescent probe to the cavity does not have to be adopted in addition to a therapeutic scope. Consequently, a burden on a patient can be significantly reduced.

Additionally, a large number of channels 66 are opened along the circumferential direction of the applicator 42, so that the fluorescent probe can be evenly sprayed over the affected area. Consequently, it can be more efficiently determined whether the affected area is present.

Furthermore, since the optical filter 68 is provided in the applicator 42, the clear observation image can be generated and displayed in the monitor device 10 while effects of the excitation light can be effectively eliminated, without mounting any sophisticated and expensive image processing software in the processor device 8.

Additionally, since the optical element 70 is movable by the adjustment wire 60, the optical filter 68 can easily adjust the amount of the excitation light to be dimmed or switch whether to cut off the excitation light. Thus, the filter adjustment is performed, so that depending on usage of the applicator device 1, the effects of the excitation light can be more effectively eliminated, and the clear observation image can be obtained.

In addition, since the applicator device 1 comprises the power source device 44 and the control circuit 46, the excitation light signal control system 74 can be provided independently of the endoscope system 2. The excitation light signal control system 74 is a system that complements a function of the optical filter 68 and that is specialized in the fluorescence observation. Therefore, even if any sophisticated and expensive image processing software is not mounted in the processor device 8, the effects of the excitation light can be effectively eliminated, and the clear observation image can be displayed in the monitor device 10.

The embodiment of the present invention has been described above, and the present invention is not limited to the above embodiment, and can be variously modified without departing from gist of the present invention.

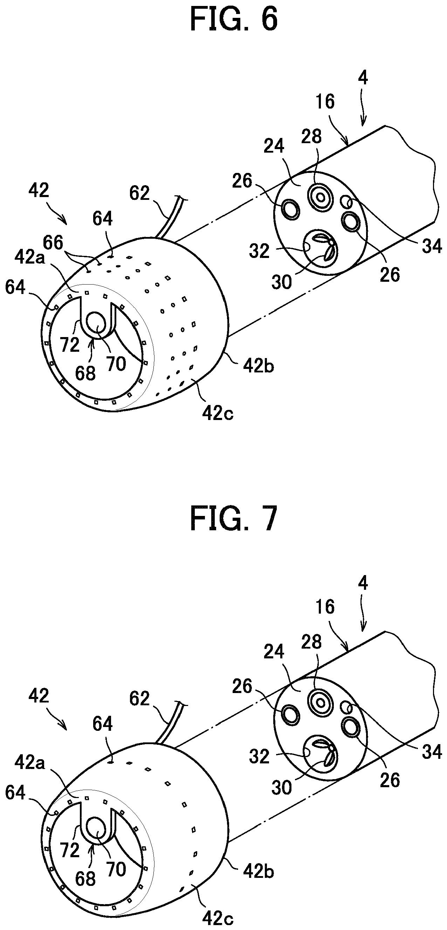

For example, as shown in FIG. 6, the LED chips 64 and channels 66 provided in the tip surface 42a of the applicator 42 are not restrictive, and may be arranged and opened in the circumferential portion 42c of the applicator 42. Consequently, a broader area in the body cavity can be irradiated with the excitation light by the respective LED chips 64. Furthermore, the fluorescent probe can be evenly sprayed from the respective channels 66 over the broader area in the body cavity.

In this case, as shown in FIG. 6, it is preferable that the respective LED chips 64 and the respective channels 66 are arranged and opened in separate annular arrays along the circumferential direction of the circumferential portion 42c. Consequently, the irradiation with the excitation light by the respective LED chips 64 and the spraying of the fluorescent probe from the respective channels 66 can be performed without any mutual interference. Furthermore, the circumferential portion 42c may be formed in a stepwise manner so that the respective LED chips 64 are arranged and opened in the annular array that is separate from the annular array of the respective channels 66, along the circumferential direction.

Alternatively, the respective LED chips 64 can be arranged by mounting the respective LED chips 64 on an unshown sheet-like flexible substrate and attaching this flexible substrate to the applicator 42. Furthermore, there are not any special restrictions on the number of the LED chips 64 or the number of the channels 66, and at least one of the chips and at least one of the channels may only be formed in the applicator 42, respectively.

Furthermore, filter adjustment means other than the above mechanism of the embodiment in which the adjustment wire 60 is used may be used, as long as the amount of the excitation light to be dimmed can be adjusted or it can be switched whether to cut off the excitation light.

Additionally, the power source switch 54 and the supply switch 58 described in the above embodiment may be arranged in the operation unit 18 of the endoscope 4 and configured to be operable with the operation of the operation unit 18. Consequently, operability of the applicator device 1 can be further improved.

In addition, the excitation light signal control system 74 is the system that complements the above described function of the optical filter 68. Therefore, the excitation light signal control system 74 does not necessarily have to be provided as long as the excitation light can be sufficiently dimmed or cut off only by the optical filter 68. Alternatively, in place of the optical filter 68, the excitation light signal control system 74 may only be provided. Furthermore, the control circuit 46 may be interposed in the cable 14 connecting the processor device 8 to the monitor device 10, to construct the excitation light signal control system 74.

Furthermore, in case where the excitation light signal control system 74 is not provided, the control circuit 46 is unnecessary, and the electric connection between the power source device 44 and the processor device 8 is also unnecessary. Therefore, in case where the excitation light signal control system 74 is not provided, simpler power supply means for the respective LED chips 64, for example, a small battery to be charged to supply power to the respective LED chips 64 may be contained in the applicator 42, in place of the power source device 44. This can achieve the applicator device 1 having more excellent portability.

Additionally, in the above embodiment, the applicator 42 also serves as the tip hood possessing the cylindrical shape. However, this is not restrictive, and there are not any special restrictions on a shape of the applicator 42 as long as at least the LED chips 64 as well as preferably the channels 66 are formed and the function of the applicator 42 can be achieved without interfering with the function of the endoscope 4. The applicator may be, for example, an attachment to be attached for use to the insertion part 16.

Furthermore, the fluorescent probe is not limited to the fluorescent probe described in the above embodiment, and the applicator device 1 can use a broad range of luminescent probes other than the fluorescent probe and including a phosphorescent probe. In this case, luminescence observation that is not limited to fluorescence and includes phosphorescence can be simply performed.

Additionally, as shown in FIG. 7, the applicator 42 may be formed in which the respective LED chips 64 are only arranged and the respective channels 66 are not provided. In this case, the applicator 42 can be used in a photodynamic therapy (PDT). The PDT is a therapeutic method of administering a photosensitive substance having tumor affinity to a living body, and then irradiating an affected area with excitation light, to selectively cause damage only to the affected area of a cancer cell or the like, thereby destroying the area.

In this case, the respective LED chips 64 emits the excitation light that excites photosensitive substances accumulated in the affected area of a biological tissue in a body cavity to cause damage to the affected area by a photochemical reaction. In the PDT, instead of spraying the luminescent probe including the fluorescent probe from the respective channels 66 into the body cavity, for example, a medicine containing the photosensitive substance is injected into a blood vessel and administered to the living body.

Furthermore, a light emitting diode other than the LED chips 64, for example, an organic EL or the like may be provided as a light emitting element in the applicator 42.

### Explanation of Reference Signs

1 applicator device for endoscope
2 endoscope system
4 endoscope
6 light source device
8 processor device
10 monitor device
16 insertion part
24 tip portion
26 illuminating section
28 light receiving section
30 treatment instrument
32 forceps opening
42 applicator
44 power source device
46 control circuit (control means)
54 power source switch (irradiation start means)
58 supply switch (supply start means)
60 adjustment wire (filter adjustment means)
64 LED chip (light emitting element or light emitting diode)
66 channel
68 optical filter

## Claims

1. An applicator device for an endoscope including an applicator attachable to and removable from an insertion part of the endoscope to be inserted into a body cavity, wherein the applicator comprises a light emitting element that irradiates an affected area of a biological tissue in the body cavity with excitation light that excites a photosensitive substance administered to the affected area so that the photosensitive substance acts on the affected area.

2. The applicator device for the endoscope according to claim 1, wherein a plurality of light emitting elements are arranged along a circumferential direction of the applicator.

3. The applicator device for the endoscope according to claim 1 or 2, wherein the applicator comprises a channel to administer the photosensitive substance to the biological tissue in the body cavity.

4. The applicator device for the endoscope according to claim 3, wherein a plurality of channels are opened along a circumferential direction of the applicator.

5. The applicator device for the endoscope according to claim 3 or 4, wherein the endoscope includes, in a tip portion of the insertion part,
an illuminating section that illuminates the body cavity with illumination light,
a light receiving section that receives light from the body cavity, and
a forceps opening that a treatment instrument inserted into the insertion part enters and exits.

6. The applicator device for the endoscope according to claim 5, wherein the applicator comprises an optical filter that covers the light receiving section to dim or cut off at least the excitation light.

7. The applicator device for the endoscope according to claim 6, wherein the applicator includes filter adjustment means for adjusting an amount of the excitation light to be dimmed or switching whether to cut off the excitation light, by the optical filter.

8. The applicator device for the endoscope according to any one of claims 5 to 7, the endoscope being included in an endoscope system comprising:
a light source device that generates the illumination light to guide the light to the illuminating section,
a processor device that processes a captured image based on light received from the light receiving section to generate an observation image, and
a monitor device electrically connected to the processor device, to display the observation image generated by the processor device,
the applicator device for the endoscope further comprising:
a power source device that supplies power to the light emitting element and is electrically connected to the processor device, and
control means for attenuating or cutting off an output signal concerning the excitation light from the processor device to the monitor device, when the power source device supplies the power to the light emitting element.

9. The applicator device for the endoscope according to any one of claims 1 to 8, wherein the applicator is a tip hood mounted to a tip portion of the insertion part.

10. The applicator device for the endoscope according to any one of claims 1 to 9, wherein the photosensitive substance is a luminescent probe, and
the light emitting element irradiates the luminescent probe administered to the affected area of the biological tissue in the body cavity by the applicator with excitation light that excites the luminescent probe to emit light.

11. The applicator device for the endoscope according to claim 10, comprising:
supply start means for starting supplying the photosensitive substance through the channel,
irradiation start means for starting the irradiation with the excitation light from the light emitting element, and
determination means for determining whether a residual affected area is present in the biological tissue based on a result of luminescence observation of the luminescent probe with the light received from the light receiving section, when after treating the affected area in the biological tissue with the treatment instrument, starting supplying the luminescent probe by the supply start means, and starting the irradiation with the excitation light by the irradiation start means.

12. The applicator device for the endoscope according to claim 10 or 11, wherein the luminescent probe is a fluorescent probe.

13. The applicator device for the endoscope according to any one of claims 1 to 9, wherein the light emitting element irradiates the affected area of the biological tissue in the body cavity with the excitation light that excites the photosensitive substance accumulated in the affected area to cause damage to the affected area by a photochemical reaction.

14. The applicator device for the endoscope according to any one of claims 1 to 13, wherein the light emitting element is a light emitting diode.
